# EUROPÄISCHE PATENTSCHRIFT

(11) **EP 0 458 297 B1**
(45) Veröffentlichungstag und Bekanntmachung des Hinweises auf die Patenterteilung: **13.03.1996**
(21) Anmeldenummer: 91108267.5
(22) Anmeldetag: 22.05.1991
(51) Int. Cl.: A61N 2/02

(54) **Applikatorspule für ein elektromagnetisches Therapiegerät**
Treatment coil for magnetotherapy apparatus
Bobine de traitement pour appareil de magnétothérapie

(30) Priorität: 22.05.1990 DE 9005821 U
(43) Veröffentlichungstag der Anmeldung: 27.11.1991
(73) Patentinhaber: Kraus, Werner, Dipl.-Ing., D-80333 München (DE)
(72) Erfinder: Kraus, Werner, Dipl.-Ing., D-80333 München (DE)
(74) Vertreter: von Bezold, Dieter, Dr.

(56) Entgegenhaltungen:
- DD-A- 236 454
- DE-A- 3 205 048
- DE-U- 8 630 652
- FR-A- 2 290 224
- US-A- 4 456 001

## Beschreibung

Die vorliegende Erfindung betrifft eine Applikatorspule mit im wesentlichen elliptischem oder länglichrundem Querschnitt für ein elektromagnetisches Therapiegerät zur Behandlung mit niederfrequenten Magnetfeldern.

Eine solche Applikatorspule ist aus DE-A-24 32 493 bekannt. Die bekannte Applikatorspule ist eine mehrlagige Zylinder- oder Solenoidspule mit einem länglichrunden, insbesondere ellipsenförmigen Querschnitt.

Die Applikatorspulen der hier interessierenden Art dienen zur Heilbehandlung mittels niederfrequenter magnetischer Wechselfelder, deren Frequenz vorzugsweise unter 20 Hz liegt. Das Magnetfeld kann unmittelbar zur Behandlung dienen oder zur Induktion von niederfrequenten Wechselströmen in einer Aufnehmerspule verwendet werden, die einen Teil einer Vorrichtung zur Förderung des Knochen- bzw. Gewebewachstums bildet (siehe z.B. DE-C-19 18 299 und CH-A-551,201).

Die gattungsgemäßen Applikatorspulen mit elliptischem Querschnitt dienen in erster Linie zur Behandlung des Rumpfbereiches des menschlichen Körpers, insbesondere des Becken- und des Schulterbereiches.

Nachteilig an den bekannten gattungsgemäßen Applikatorspulen ist es, daß sie vom Patienten bei längeren Behandlungszeiten und/oder empfindlicher Konstitution als unbequem empfunden werden.

Die vorliegende Erfindung löst, ausgehend von diesem Stand der Technik, die Aufgabe, eine Applikatorspule für ein elektromagnetisches Therapiegerät, die einen im wesentlichen länglichrunden Querschnitt hat, so auszubilden, daß sie auch von empfindlichen Patienten oder bei längerdauernder Behandlung nicht als unbequem empfunden wird, dadurch, daß der Querschnitt bezüglich des großen Durchmessers unsymmetrisch ist.

Dadurch, daß der eine, bei Gebrauch untere Teil der Spule eine geringere Krümmung hat, also flacher ist, als der andere, im Gebrauch obere Teil, wird die Spule vom Patienten praktisch nicht mehr als störend empfunden.

Die Erfindung wird im folgenden unter Bezugnahme auf die Zeichnungen näher erläutert. Es zeigen:
- Figur 1: eine Stirnansicht einer Applikatorspule gemäß einer Ausführungsform der Erfindung und
- Figur 2: eine Draufsicht auf die Applikatorspule gemäß Figur 1.

Die in der Zeichnung dargestellte Applikatorspule 10 hat die Form eines kurzen, geraden Zylinders und enthält eine mehrlagige Drahtwicklung (nicht dargstellt), die in bekannter Weise mit Kunststoff ummantelt und mit Anschlußklemmen versehen ist, die sich in einem an der Seite der Spule angebrachten Anschlußkasten 12 befinden. Die Applikatorspule ist in bekannter Weise für die Erzeugung niederfrequenter Magnetfelder bemessen, insbesondere von Magnetfeldern mit Frequenzen von 20 Hz und darunter.

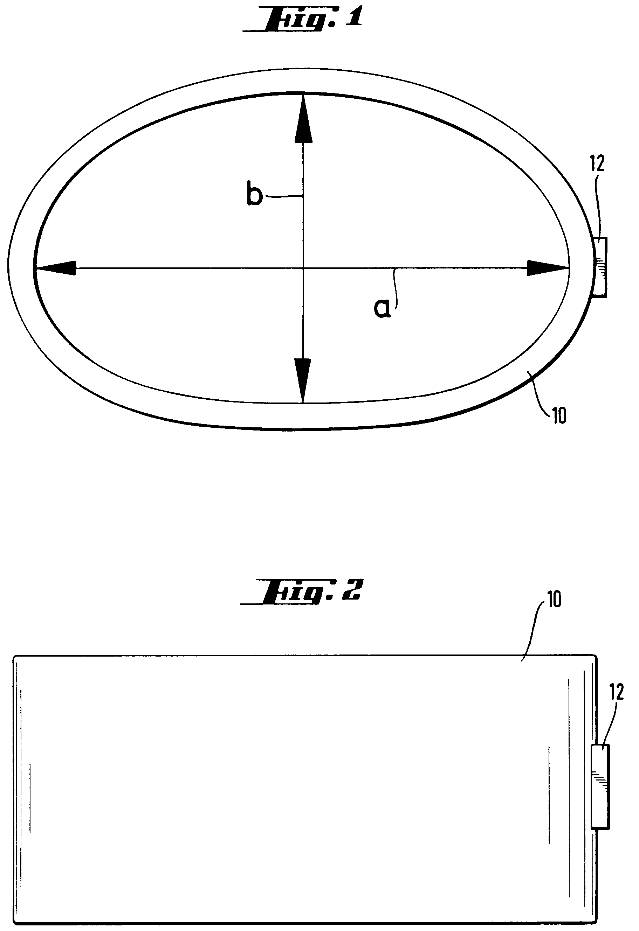

Wie Figur 1 zeigt, hat die Spule einen länglichrunden Querschnitt mit einem großen Durchmesser a und einem kleinen Durchmesser b. Bezüglich des kleinen Durchmessers b ist der Querschnitt symmetrisch. Bezüglich des großen Durchmessers a ist der Querschnitt jedoch erfindungsgemäß unsymmetrisch. Der bei Gebrauch und in Figur 1 untere Teil der Spule hat die Form einer flachen Schale und im mittleren Bereich eine geringere Krümmung oder Wölbung als der obere Teil. Der untere und der obere Teil der Spule können jeweils einen Querschnitt in Form einer Hälfte einer durch ihre große Achse geteilten Ellipse haben, wobei das Verhältnis der Länge der kleinen Achse zur Länge der großen Achse beim unteren Teil kleiner ist als beim oberen Teil. Dementsprechend hat der Teil des Querschnitts, der sich unterhalb des großen Durchmesses a befindet, eine kleinere Fläche als der sich oberhalb des großen Durchmessers a befindende Teil.

Durch die flachere Wölbung paßt sich der untere Teil der Spule besser an die Form des von der Spule umgebenden Teiles des Rumpfes des Patienten an, insbesondere den Becken- oder Schulterbereich, so daß die Spule nicht als störend empfunden wird. Die stärkere Krümmung und die größere Querschnittsfläche im oberen Teil der Spule läßt Raum für den Bauch und für die Brust.

## Patentansprüche

1. Applikatorspule für ein elektromagnetisches Therapiegerät mit einem im wesentlichen länglichrunden Querschnitt, der einen großen und einen kleinen Durchmesser (a bzw. b) hat, **dadurch gekennzeichnet**, daß der Querschnitt bezüglich des großen Durchmessers (a) unsymmetrisch ist.

2. Applikatorspule nach Anspruch 1, **dadurch gekennzeichnet**, daß der bei Gebrauch untere Teil der Spule eine geringere Wölbung aufweist als der obere Teil.

3. Applikatorspule nach Anspruch 1 oder 2, **dadurch gekennzeichnet**, daß sie die Form eines kurzen geraden Zylinders hat.

4. Applikatorspule nach einem der Ansprüche 1 bis 3, **dadurch gekennzeichnet**, daß sie eine mehrlagige, mit einer Ummantelung versehene Drahtwicklung aufweist.

5. Applikatorspule nach einem der Ansprüche 1 bis 4, **dadurch gekennzeichnet**, daß sie für Frequenzen von 20 Hz und darunter bemessen ist.

## Claims

1. Treatment coil for a magnetotherapy apparatus, said coil having an essentially oblong-round cross-section having a large and a small diameter (a, b, respectively), **characterized in that** the cross-section is assymetrical with respect to the large diameter (a).

2. Treatment coil according to claim 1, **characterized in that** the portion of the coil which is the lower one during use, has a smaller arching than the upper portion.

3. Treatment coil according to claim 1 or 2, **characterized in that** it has the shape of a short straight cylinder.

4. Treatment coil according to any of claims 1 to 3, **characterized in that** it comprises a multi-layer wire winding provided with a casing.

5. Treatment coil according to any of claims 1 to 4, **characterized in that** it is dimensioned for frequencies of 20 Hz and below.

## Revendications

1. Bobine de traitement pour un appareil de magnétothérapie avec un coupe transversale oblonge-ronde que a un grande et un petit diamètre (a ou b), characterisée en que la coupe transversale est asymétrique relatif à le grand diamètre (a).

2. Bobine de traitement selon revendication 1, characterisée en que le part qui est en usage en bas a un bombement plus faible que le part supérieur.

3. Bobine de traitement selon les revendicatins 1 ou 2, characterisée en que elle a la forme d'un court cylindre droit.

4. Bobine de traitement selon un des revendications 1 à 3, characterisée en que elle a un enroulement en fils à plusieurs couches muni avec un enrobage.

5. Bobine de traitement selon un des revendications 1 à 4, characterisée en que elle est dimensionée pour fréquences de 20 Hz et là-dessous.
